# DEMANDE DE BREVET EUROPEEN

(11) **EP 3 042 662 A1**
(43) Date de publication de la demande: **13.07.2016**
(21) Numéro de dépôt: 16305025.5
(22) Date de dépôt: 12.01.2016
(51) Int. Cl.: A61K 36/84, A61Q 19/08, A61K 36/28, A61K 36/31

(54) **EXTRAITS DE SALADE, COMPOSITIONS ET UTILISATIONS**

(30) Priorité: 12.01.2015 FR 1550219
(71) Demandeur: BIOLIE, 54003 Nancy Cedex (FR)
(72) Inventeur: CLAISSE, Nathalie, 54000 Nancy (FR); RICOCHON, Guillaume, 54270 Essey-Lès-Nancy (FR); ATTENOT, Nicolas, 54000 Nancy (FR)
(74) Mandataire: Icosa

(57) **Abrégé**

La présente invention concerne un extrait de feuilles de salades de la famille des *Asteraceae,* de la famille des *Brassicaceae* et de la famille des *Valerianaceae,* et en ce que l'extrait comprend de 0.01 à 25% de composés polyphénoliques en poids par rapport au poids de l'extrait sec ; l'invention concerne également une composition comprenant l'extrait de l'invention, et l'utilisation cosmétique, dermocosmétique, cosméceutique ou dermatologique de l'extrait de l'invention.

## Description

### DOMAINE DE L'INVENTION

La présente invention concerne le domaine de la cosmétique, de la dermocosmétique, de la cosméceutique et/ou de la dermatologie. Plus particulièrement, l'invention s'intéresse à des extraits de salades, notamment de la famille des *Asteraceae,* de la famille des *Brassicaceae* et de la famille des *Valerianaceae*, et à leur utilisation en tant qu'actifs cosmétique ou dermocosmétique ou cosméceutique ou en tant que principes actifs dermatologiques.

### ÉTAT DE LA TECHNIQUE

Dans les différents organes des plantes, des substances naturelles souvent à haute valeur ajoutée sont synthétisées. Depuis de nombreuses années, l'étude des plantes a démontré qu'elles contiennent de nombreuses substances actives jusque-là synthétisées chimiquement, à l'état naturel. En outre de nombreux actifs ou principes actifs contenus dans les plantes n'ont encore jamais été synthétisés, et restent donc exclusivement disponibles dans le végétal. Dans une démarche écologique et naturelle, de nombreux produits cosmétiques sont aujourd'hui fabriqués à base d'extraits de plantes et d'actifs naturels. Par exemple, les propriétés antioxydantes du thé sont désormais reconnues et utilisées dans de nombreux produits.

L'industrie cosmétique, comme l'industrie dermatologique, sont aujourd'hui à la recherche de nouveaux moyens permettant de concevoir des produits alliant efficacité, innovation et écologie, en utilisant notamment des extraits naturels de plantes.

La Demanderesse a développé une technologie d'extraction enzymatique à partir de matière végétale (WO 2011/045387) et s'intéresse tout particulièrement aux propriétés des produits extraits issus de ce procédé. Cette technologie combine des avantages technologiques, économiques, règlementaires et environnementaux. En particulier, la Demanderesse a appliqué cette technologie à une source végétale naturelle, qui est un mélange de salades, et a obtenu des extraits d'intérêt en cosmétique, dermocosmétique, cosméceutique et dermatologie.

Dans l'art antérieur, d'autres procédés d'extraction ont été décrits. Par exemple, le brevet EP 1362021 décrit un procédé d'extraction de composés issus de plantes de la famille des Composées ou de la famille des Lilliacées comprenant une étape d'extraction pour obtenir un extrait brut, puis une étape d'adsorption sur une résine absorbante particulière, suivie d'une étape d'élution et enfin de concentration. Ce procédé, et en particulier l'étape d'adsorption, ne permet pas d'obtenir un extrait parfaitement naturel, c'est-à-dire exempt de toute trace de produits chimiques qui pourraient provenir du procédé d'extraction.

Il en va de même pour le procédé décrit dans le brevet FR 2626469, qui s'intéresse aux propriétés anti-radicalaires d'un extrait des parties aériennes de la salade pain de sucre.

Par ailleurs, la crème pour les mains et le corps proposée par Portugal Cosmetics Lechuga (Lechuga Fluida) contient un extrait de salade mais uniquement de laitue. Il en est de même d'autres crèmes pour rendre la peau lisse, dans lesquelles une seule variété de salade est utilisée.

D'autre part, la Demanderesse a constaté de manière inattendue et surprenante que ces extraits de salade présentent simultanément des propriétés anti-inflammatoires, inhibitrices de la collagénase, et lissantes, qui n'avaient jamais été reportées dans l'art antérieur et en font des extraits d'un intérêt nouveau pour la fabrication de produits cosmétiques, dermocosmétiques, cosméceutiques ou dermatologiques.

### RESUMÉ

Ainsi, dans un premier aspect, l'invention concerne de nouveaux extraits de salade, de préférence de la famille des *Asteraceae,* de la famille des *Brassicaceae* et/ou, préférentiellement et, de la famille des *Valerianaceae*, en tant qu'actifs ou principes actifs pour la préparation de compositions cosmétiques, dermocosmétiques, cosméceutiques ou dermatologiques.

Dans un mode de réalisation préféré, l'invention a pour objet un extrait de feuilles de salades, réalisé à partir d'un mélange de salades de la famille des *Asteraceae,* de la famille des *Brassicaceae* et de la famille des *Valerianaceae*, ledit extrait comprenant de 0.01 à 25% de composés polyphénoliques en poids par rapport au poids de l'extrait sec.

De préférence, le mélange est un mélange de salades de la famille des *Asteraceae* du genre *Lactuca*, de préférence de l'espèce *Lactuca sativa*, de préférence des variétés *capitata, crispa, longifolia ;* de salades de la famille des *Brassicaceae* du genre *Eruca,* de préférence la variété *Eruca sativa ;* et de salades de la famille des *Valerianaceae* du genre *Valerianella,* de préférence la variété *Valerianella locusta*.

Suivant un mode de réalisation, l'extrait selon l'invention comprend, parmi les composés polyphénoliques, de 1 à 30% d'acide caféoyltartarique en poids par rapport au poids total des composés polyphénoliques. Suivant un mode de réalisation, l'extrait selon l'invention comprend, parmi les composés polyphénoliques, de 6 à 30% d'acide caféique en poids par rapport au poids total des composés polyphénoliques. Selon un mode de réalisation, l'extrait selon l'invention comprend, parmi les composés polyphénoliques, de 0 à 10% d'acide chlorogénique en poids par rapport au poids total des composés polyphénoliques. Selon un mode de réalisation, l'extrait selon l'invention comprend, parmi les composés polyphénoliques, de 0 à 10% d'acide chicorique en poids par rapport au poids total des composés polyphénoliques.

Suivant un mode de réalisation préféré, l'extrait selon l'invention est susceptible d'être obtenu par broyage des parties destinées à la préparation de l'extrait, qui sont préférentiellement les feuilles des salades, suivi d'une hydrolyse enzymatique dudit broyat, et optionnellement d'une évaporation d'eau. Selon un mode de réalisation, l'extrait selon l'invention a de 1 à 10% de matière sèche, en poids par rapport au poids total de l'extrait. Dans un aspect particulier, l'invention concerne un extrait concentré obtenu par évaporation de l'eau d'un extrait susceptible d'être obtenu par le procédé de broyage suivi d'hydrolyse enzymatique selon l'invention. Selon un mode de réalisation, le concentré selon l'invention a de plus de 10 à 99,9% de matière sèche par rapport au poids total de l'extrait concentré.

Dans un second aspect, l'invention a pour objet une composition comprenant un extrait ou un extrait concentré selon l'invention, en association avec au moins un excipient cosmétiquement, dermatologiquement, dermocosmétiquement acceptable. Avantageusement, la composition comprend de 0.5 à 5%, de préférence de 1 à 3% d'extrait ou d'extrait concentré selon l'invention, en poids par rapport au poids total de la composition. Suivant un mode de réalisation, la composition comprend au moins un autre actif cosmétique, dermocosmétique, cosméceutique ou dermatologique.

Suivant un mode de réalisation, la composition est formulée, c'est-à-dire mise en association avec différents excipients, pour être administrée topiquement sur la peau du visage et/ou du corps. Suivant un autre mode de réalisation, la composition est formulée pour être administrée par voie orale.

Dans un troisième aspect, l'invention a pour objet un actif cosmétique, dermocosmétique ou cosméceutique ou un principe actif dermatologique caractérisé en ce qu'il comprend un extrait ou une composition selon l'invention.

Dans un quatrième aspect, l'invention a pour objet l'utilisation d'un extrait, d'un extrait concentré, ou une composition selon l'invention en tant qu'agent anti-inflammatoire et/ou apaisant ; l'utilisation d'un extrait, d'un extrait concentré, ou une composition selon l'invention en tant qu'agent lissant ; et/ou l'utilisation d'un extrait, d'un extrait concentré, ou une composition selon l'invention en tant qu'agent inhibiteur de collagénase.

Dans un cinquième aspect, l'invention a pour objet une méthode de soin non-thérapeutique de la peau, de préférence une méthode cosmétique, dermocosmétique ou cosméceutique pour prévenir, retarder et/ou lutter contre le photo-vieillissement, pour prévenir et/ou atténuer les rides et/ou ridules, pour réduire et/ou limiter les vergetures, comprenant l'administration topique ou orale d'un extrait selon l'invention ou d'une composition selon l'invention.

Dans un sixième aspect, l'invention a pour objet une composition pharmaceutique, en particulier dermatologique, ou un médicament, comprenant un extrait ou une composition selon l'invention.

### DÉFINITIONS

Dans la présente invention, les termes ci-dessous sont définis de la manière suivante :
- Le terme « **extrait** » concerne une préparation liquide, semi-solide ou solide, obtenue par extraction de plantes potagères, en particulier de salades. Selon un mode de réalisation selon l'invention, l'extrait est aqueux. Selon un autre mode de réalisation selon l'invention, l'extrait est sec. De préférence, l'extraction est biologique, très préférentiellement enzymatique.
- Le terme « **polyphénols** » concerne des molécules possédant un ou plusieurs cycles benzéniques portant une ou plusieurs fonctions hydroxyles. Dans un mode de réalisation, les polyphénols de l'invention sont des molécules portant plusieurs fonctions hydroxyle phénolique.
- Le terme « **polyphénols glycosylés** » désigne des polyphénols liés de façon covalente à un glucide.
- Le terme « **actif** » concerne un ingrédient qui possède une activité précise à l'origine de l'efficacité du produit cosmétique, dermocosmétique ou cosméceutique.
- Le terme « **cosméceutique** » se réfère à un produit cosmétique à administration orale.
- Le terme « **principe actif** » concerne une substance entrant dans la composition d'un médicament, et lui conférant ses propriétés thérapeutiques.
- Le terme « **anti-inflammatoire** » concerne une substance qui s'oppose ou qui combat une inflammation. Un anti-inflammatoire peut être utilisé dans le traitement local de l'inflammation ou le traitement général des maladies inflammatoires.
- Le terme « **inhibiteur de collagénase** » concerne une substance ayant l'effet de réduire ou de réguler négativement l'activité biologique d'une enzyme de la famille des collagénases.
- Le terme « **lissant** » concerne une substance qui réduit les irrégularités de la peau.
- Le terme « **apaisant** » concerne une substance qui aide à réduire l'inconfort de la peau.
- Précédant une valeur chiffrée, le terme « **environ** » signifie plus ou moins 10% de ladite valeur.

### DESCRIPTION DÉTAILLÉE

### Extrait

La présente invention concerne un extrait issu de plantes potagères connues sous le terme générique de salades.

Selon un mode avantageux de l'invention, l'extrait est issu, ou susceptible d'être obtenu, à partir d'un mélange de salades.

Selon un mode de réalisation, les plantes potagères dont est issu l'extrait de l'invention comprennent les plantes, et en particulier les salades, de la famille des *Asteraceae,* de la famille des *Brassicaceae,* et/ou de la famille des *Valerianaceae.*

Selon un mode de réalisation, les plantes de la famille des *Asteraceae* sont du genre *Lactuca*, de préférence de l'espèce *Lactuca sativa*. Parmi les différentes variétés de *Lactuca sativa*, on citera par exemple les variétés *capitata, crispa, longifolia*.

Comme exemples de plantes de la famille des *Brassicaceae,* on peut citer les plantes du genre *Eruca,* notamment la variété *Eruca sativa*.

Parmi les plantes de la famille des *Valerianaceae,* on citera les plantes du genre *Valerianella,* notamment la variété *Valerianella locusta*.

Suivant un mode de réalisation particulier, le mélange est un mélange de *Lactuca*, *Eruca,* et *Valerianella*.

Selon un mode de réalisation, les plantes potagères dont est issu l'extrait de l'invention peuvent comprendre en outre des plantes de la famille des *Chenopodiaceae*. Comme exemples de plantes de la famille des *Chenopodiaceae,* on peut citer les plantes du genre *Spinacia*, et notamment la variété *Spinacia oleracea.* Selon un autre mode de réalisation, l'extrait de l'invention est issu de plantes potagères ne comprenant pas de plantes de la famille des *Chenopodiaceae*.

Selon un mode de réalisation, l'extrait de l'invention est issu de plantes potagères comprenant des plantes du genre *Cichorium.* Selon un autre mode de réalisation, l'extrait de l'invention est issu de plantes potagères ne comprenant pas de plantes du genre *Cichorium.*

Selon un mode de réalisation, l'extrait de l'invention est obtenu à partir d'un mélange de salades comprenant de 25 à 40% de salades de la famille des *Asteraceae,* de préférence de 30 à 35% ; de 25 à 40% de salades de la famille des *Brassicaceae,* de préférence de 30 à 35% ; et de 25 à 40% de salades de la famille des *Valerianaceae,* de préférence de 30 à 35%. Selon un mode particulier de réalisation, l'extrait de l'invention est obtenu à partir d'un mélange de salades comprenant environ 33% de salades de la famille des *Asteraceae,* environ 33% de salades de la famille des *Brassicaceae,* et environ 33% de salades de la famille des *Valerianaceae.*

Selon un autre mode de réalisation, l'extrait de l'invention est obtenu à partir d'un mélange de salades comprenant de 90 à 100% de salades de la famille des *Asteraceae,* de préférence de 95 à 99% ; de 0 à 10% de salades de la famille des *Brassicaceae,* de préférence de 1 à 5% ; et de 0 à 10% de salades de la famille des *Valerianaceae,* de préférence de 1 à 5%. Selon un mode particulier de réalisation, l'extrait de l'invention est obtenu à partir d'un mélange de salades comprenant environ 98% de salades de la famille des *Asteraceae,* environ 1% de salades de la famille des *Brassicaceae,* et environ 1% de salades de la famille des *Valerianaceae.*

Dans la présente invention, l'expression « extrait issu de plantes potagères » signifie que l'extrait peut provenir de toutes les parties constituant lesdites plantes potagères. Selon un mode de réalisation, l'extrait de l'invention est issu des parties aériennes, i.e. des feuilles et/ou des fleurs, desdites plantes potagères. Selon un mode de réalisation, l'extrait de l'invention est issu des feuilles des plantes potagères. Selon un mode de réalisation, l'extrait de l'invention est issu de feuilles de salades, de préférence de feuilles d'un mélange de salades.

Selon un autre mode de réalisation, l'extrait de l'invention est issu des parties souterraines, i.e. des racines, desdites plantes potagères. Selon un mode de réalisation, l'extrait de l'invention n'est pas issu des parties souterraines des plantes potagères.

Selon un mode de réalisation, l'extrait de l'invention comprend des composés polyphénoliques. Selon un mode avantageux de réalisation, l'extrait de l'invention comprend de 0.01 à 25%, de préférence de 0.5 à 25%, de préférence de 1 à 25% de composés polyphénoliques en poids par rapport au poids total de l'extrait sec. Selon un mode de réalisation, la teneur en composés polyphénoliques de l'extrait de l'invention est inférieure à 25% en poids par rapport au poids total de l'extrait sec. Selon un mode de réalisation, la teneur en composés polyphénoliques de l'extrait de l'invention est supérieure à 1% en poids par rapport au poids total de l'extrait sec. Selon un mode de réalisation, l'extrait de l'invention comprend de 1 à 5% de composés polyphénoliques en poids par rapport au poids total de l'extrait sec.

Selon un mode de réalisation, parmi les composés polyphénoliques de l'extrait de l'invention, au moins 1% en poids par rapport au poids des composés polyphénoliques sont des composés polyphénoliques glycosylés. Ainsi, selon un mode de réalisation, l'extrait de l'invention comprend au moins 0.0001%, de préférence au moins 0.005%, de préférence au moins 0.01% de composés polyphénoliques glycosylés en poids par rapport au poids total de l'extrait sec.

Selon un mode de réalisation, les composés polyphénoliques de l'extrait de l'invention comprennent de l'acide caféoyltartarique. Selon un mode de réalisation, l'extrait de l'invention comprend de 1 à 30% d'acide caféoyltartarique en poids par rapport au poids total des composés polyphénoliques de l'extrait, de préférence de 5 à 20%, plus préférentiellement de 6 à 15%. Ainsi, selon un mode de réalisation, l'extrait de l'invention comprend de 0.0001 à 7.5% d'acide caféoyltartarique en poids par rapport au poids total de l'extrait sec.

Selon un mode de réalisation, les composés polyphénoliques de l'extrait de l'invention comprennent de l'acide caféique. Selon un mode de réalisation, l'extrait de l'invention comprend de 6 à 30% d'acide caféique en poids par rapport au poids total des composés polyphénoliques de l'extrait. Selon un mode de réalisation, l'extrait de l'invention comprend de 1 à 5% d'acide caféique en poids par rapport au poids total de l'extrait sec.

Selon un mode de réalisation, les composés polyphénoliques de l'extrait de l'invention comprennent de l'acide chlorogénique. Selon un mode de réalisation, l'extrait de l'invention comprend de 0 à 10% d'acide chlorogénique en poids par rapport au poids total des composés polyphénoliques de l'extrait. Selon un mode de réalisation, l'extrait de l'invention comprend de 0 à 2% d'acide chlorogénique en poids par rapport au poids total de l'extrait sec.

Selon un mode de réalisation, les composés polyphénoliques de l'extrait de l'invention comprennent de l'acide chicorique. Selon un mode de réalisation, l'extrait de l'invention comprend de 0 à 10% d'acide chicorique en poids par rapport au poids total des composés polyphénoliques de l'extrait. Selon un mode de réalisation, l'extrait de l'invention comprend de 0 à 2% d'acide chicorique en poids par rapport au poids total de l'extrait sec.

### Procédé

L'extrait selon l'invention peut être obtenu par toute méthode d'extraction connue de l'homme du métier. Toutefois, la Demanderesse privilégie les procédés d'extraction permettant d'obtenir des produits propres, c'est-à-dire exempts de trace de produits chimiques provenant du procédé d'extraction lui-même. Selon un mode de réalisation préféré, l'extrait selon l'invention est obtenu par un procédé d'extraction biologique, de préférence enzymatique.

Ainsi, l'invention concerne également un procédé de préparation de l'extrait de l'invention.

Selon un mode de réalisation, le procédé de l'invention comprend au moins une étape d'hydrolyse enzymatique. Selon un mode de réalisation, l'hydrolyse enzymatique est réalisée par un mélange enzymatique contenant au moins une cellulase, au moins une hémicellulase et au moins une pectinase.

Selon un mode de réalisation, le procédé de l'invention comprend les étapes suivantes :
a) après une étape optionnelle d'addition d'eau, broyage des feuilles de salade et addition audit broyat d'un mélange enzymatique contenant au moins une cellulase, au moins une hémicellulase et au moins une pectinase, le rapport entre l'activité de la pectinase et l'activité de la cellulase étant d'au moins 0.14, de préférence compris entre 0.3 et 2.5, et plus préférentiellement entre 0.35 et 0.45, et le rapport entre l'activité de la pectinase et l'activité de l'hémicellulase étant d'au moins 7.10⁻³, de préférence compris entre 1.10⁻² et 0.5, et plus préférentiellement entre 1.10⁻² et 2.10⁻², l'activité de la pectinase étant inférieure à 120 µmol/min/ml, et de préférence inférieure à 100 µmol/min/ml,
b) incubation sous agitation et à une température de 37 à 60°C de la matière végétale et du mélange enzymatique pour libérer dans le milieu réactionnel entre autres des phénols, des protéines et des sucres fermentescibles, pendant une durée dépendant des rendements recherchés,
c) séparation du milieu réactionnel pour obtenir une phase aqueuse contenant des molécules hydrophiles d'intérêt dont des phénols, des protéines et des sucres fermentescibles, et une phase solide,
d) optionnellement, concentration de la phase aqueuse par évaporation de l'eau afin d'obtenir un extrait dont la teneur en eau a été réduite par rapport à la phase aqueuse de l'étape c).

Selon un mode de réalisation, l'extrait selon l'invention est un extrait concentré. Dans ce mode de réalisation, l'étape d) du procédé est réalisée. Par « extrait concentré », on entend un extrait dont la teneur en eau a été réduite par rapport à l'extrait obtenu à la fin de l'étape d). La concentration de l'extrait est réalisée par l'une quelconque des méthodes connues par l'homme du métier. La réduction de la teneur en eau de l'extrait peut par exemple être réalisée par évaporation de l'eau à température ambiante, ou à une température comprise entre 60°C et 100°C à pression atmosphérique ou à une température d'ébullition de l'eau à pression réduite. La réduction de la teneur en eau de l'extrait peut également être réalisée par filtration ou ultrafiltration.

Selon un mode de réalisation, l'extrait de l'invention est concentré au moins deux fois par rapport à l'extrait aqueux obtenu à l'étape c). Selon un mode de réalisation, l'extrait de l'invention est concentré au moins 5 fois par rapport à l'extrait aqueux obtenu à l'étape c). Selon un mode avantageux de réalisation, l'extrait de l'invention est concentré 10 fois par rapport à l'extrait aqueux obtenu à l'étape c).

Selon un mode de réalisation préféré, l'extrait selon l'invention est préparé selon le procédé décrit dans la demande de brevet WO 2011/045387. En effet, la Demanderesse a montré que l'utilisation du procédé décrit dans la demande de brevet WO 2011/045387 permet d'obtenir un extrait riche en polyphénols. En particulier, l'utilisation du procédé décrit dans la demande de brevet WO 2011/045387 permet d'obtenir un extrait comprenant de 0.01 à 25% de composés polyphénoliques en poids par rapport au poids total de l'extrait sec.

### Compositions

L'invention concerne également une composition comprenant l'extrait de l'invention en tant qu'actif ou principe actif.

Dans un mode de réalisation, la composition de l'invention comprend en outre au moins un excipient approprié. Selon un mode de réalisation, la composition de l'invention est une composition cosmétique et comprend un excipient cosmétiquement acceptable. Selon un autre mode de réalisation, la composition de l'invention est une composition dermocosmétique et comprend un excipient dermocosmétiquement acceptable. Selon un autre mode de réalisation, la composition de l'invention est une composition cosméceutique et comprend un excipient cosméceutiquement acceptable. Selon un autre mode de réalisation, la composition de l'invention est une composition dermatologique et comprend un excipient dermatologiquement acceptable. Les excipients acceptables et leurs quantités sont ceux classiquement utilisés dans les domaines considérés et bien connus par l'homme du métier.

Dans un mode de réalisation, la composition de l'invention comprend une teneur en extrait de l'invention allant de 0.5 à 5%, de préférence de 1 à 3%, en poids par rapport au poids total de la composition.

Dans un mode de réalisation, la composition de l'invention peut comprendre en outre au moins un actif additionnel connu pour son action anti-inflammatoire, inhibitrice de collagénase, lissante et/ou hydratante. Selon un mode de réalisation, l'actif additionnel est choisi parmi la vitamine E, la cystéine, le sulfonate d'hydroxyquinoline, la phénantroline, le trinitrobenzène, l'acétylimidazole, ou des extraits de plantes telles que notamment le thé, la rose ou la lavande.

La composition selon l'invention peut également contenir les adjuvants habituels dans le domaine de la cosmétique, de la dermocosmétique, de la cosméceutique ou de la dermatologie, tels que les gélifiants, les épaississants, les conservateurs, les solvants, les parfums, les agents chélateurs, les absorbeurs d'odeur, les filtres chimiques ou minéraux, les pigments minéraux, les tensioactifs, les polymères et les matières colorantes. Les quantités de ces différents adjuvants sont celles classiquement utilisées dans les domaines considérés.

Selon un mode de réalisation, l'extrait ou la composition de l'invention est formulé sans ingrédient pouvant représenter un risque pour la santé (irritation, allergies, perturbation endocrinienne, de la reproduction) et/ou l'environnement. En particulier, selon un mode de réalisation, l'extrait ou la composition de l'invention ne contient pas d'éthers de glycol, tels que les dérivés de l'éthylène-glycol ou les dérivés du propylène-glycol, de parabène, de phtalate, de formaldéhyde. Selon un mode avantageux de réalisation, l'extrait ou la composition de l'invention est adapté à une administration à une femme enceinte et est compatible avec l'allaitement.

Dans un mode de réalisation, la composition de l'invention est administrée topiquement sur le visage et le corps. Selon un mode de réalisation, la composition de l'invention est avantageusement appliquée sur la peau du visage, c'est-à-dire la peau de la face, du cou, du décolleté et/ou du contour des yeux et sur la peau des mains. Selon un autre mode de réalisation, la composition de l'invention est avantageusement appliquée sur la peau du corps, en particulier de l'abdomen, des cuisses, des hanches, des fesses, des seins, des bras. La composition de l'invention peut alors être sous forme, par exemple, de gel, lait, crème, lotion, émulsion, huile, baume, onguent.

Dans un mode de réalisation, la composition de l'invention est administrée oralement. La composition de l'invention peut alors être sous forme, par exemple, de comprimés, gélules, capsules, ampoules, sirops, gouttes.

### Utilisations

La Demanderesse a mis en évidence que l'extrait de l'invention possède des propriétés anti-inflammatoires (exemple 2). Notamment, il présente des propriétés apaisantes et un effet protecteur contre l'exposition aux UVB.

Or, les UV font partie des facteurs extérieurs pouvant influencer le vieillissement de la peau. En particulier, les UVB sont la principale cause du photo-vieillissement.

L'invention concerne donc l'utilisation d'un extrait ou d'une composition selon l'invention pour protéger la peau et/ou lutter contre les rayons UV, de préférence les UVB. Selon un mode de réalisation, l'extrait ou la composition de l'invention est utile pour prévenir et/ou lutter contre le photo-vieillissement.

La Demanderesse a également mis en évidence qu'un extrait de l'invention possède une activité inhibitrice de collagénase induisant un effet tenseur de la peau (exemple 3).

Le collagène est l'une des principales protéines structurales de la peau produites par les fibroblastes. Il s'agit de la protéine la plus abondante dans un organisme humain (également la protéine la plus abondante du règne animal), représentant le quart de la masse protéique. Les principales fonctions du collagène sont entre autres de fournir une fermeté mécanique à la peau grâce à sa structure rigide en triple-hélice, amortir le tissu conjonctif, maintenir l'adhésion cellulaire. En particulier, il permet de renforcer et tendre la peau afin de la protéger des stimuli ou contraintes extérieures.

De plus, la réduction du collagène qui compte pour 90% du volume cutané est en relation étroite avec le vieillissement de la peau. Avec le temps, la peau perd en souplesse et en élasticité, et des rides se forment. La dégradation du collagène accentue ce processus et favorise l'apparition des rides et ridules.

Les collagénases sont des enzymes capables de rompre les liaisons peptidiques du collagène et sont donc responsables de sa dégradation. De plus, certaines enzymes de dégradation du collagène sont activées par une irradiation aux ultraviolets. L'inhibition de collagénases, ainsi que la protection contre les UV telle que décrite ci-dessus, est une solution pour réduire et/ou limiter la dégradation du collagène, et par conséquent l'apparition des rides et ridules.

Selon un mode de réalisation, l'extrait de l'invention ou la composition de l'invention est donc utile pour lutter contre la dégradation du collagène cutané. Selon un mode de réalisation avantageux, l'extrait de l'invention ou la composition de l'invention est utile pour prévenir et/ou atténuer les rides et/ou ridules.

Outre son effet tenseur, l'extrait de l'invention présente également des propriétés lissantes (exemple 4).

L'extrait ou la composition de l'invention est également utile pour réduire et/ou atténuer les cicatrices. Les cicatrices peuvent se trouver sur le visage ou sur le corps. Les cicatrices peuvent par exemple être atrophiques (i.e. en creux), hypertrophiques (i.e. en relief).

Selon un mode de réalisation, l'extrait ou la composition de l'invention est utile pour réduire et/ou atténuer les cicatrices d'acné. Selon un autre mode de réalisation l'extrait ou la composition de l'invention est utile pour réduire et/ou atténuer les cicatrices liées à l'âge. Selon un autre mode de réalisation l'extrait ou la composition de l'invention est utile pour réduire et/ou atténuer les cicatrices liées à une brûlure. Selon un autre mode de réalisation l'extrait ou la composition de l'invention est utile pour réduire et/ou atténuer les cicatrices liées à une coupure.

Le collagène est responsable, avec les fibres élastiques, de la souplesse de la peau. Si la production de collagène diminue, par exemple à cause d'un excès de corticoïdes dans l'organisme, la peau devient moins élastique et des vergetures peuvent se former. De même, des vergetures peuvent apparaître lorsque la peau est soumise à un étirement important, rapide, et/ou brutal. Les vergetures, ou striae distensae, sont des sortes de petites fissures semblables à des cicatrices molles formées sur la peau et sont assimilables à des cicatrices (car elles ont subi les mêmes étapes de formation qu'après un traumatisme de la peau). Leur guérison est actuellement impossible, mais une atténuation et une amélioration des lésions sont possibles. Une grossesse ou une prise de poids importante, une poussée de croissance à la puberté, mais aussi une modification hormonale sont autant de cas où l'apparition de vergetures peut survenir. Par exemple, lors de la grossesse, l'augmentation des hormones conduit à une élévation naturelle des corticoïdes, qui agissent sur le métabolisme du tissu conjonctif en limitant la synthèse de collagène et en activant sa dégradation. L'inhibition de l'activité de dégradation du collagène constitue donc un moyen de réduire et/ ou limiter les vergetures. Par ailleurs, ce processus s'accompagne d'une phase inflammatoire. En conséquence, il y a dégénérescence du tissu dermique conduisant à la formation d'une cicatrice dermique atrophique.

Grâce à ses propriétés anti-inflammatoires, inhibitrices de collagénase et lissantes, l'extrait ou la composition de l'invention est donc tout particulièrement destiné à être utile pour réduire et/ou limiter les vergetures. Selon un mode de réalisation, les vergetures sont dues à une croissance rapide. Selon un autre mode de réalisation, les vergetures sont dues à une prise de poids rapide et/ou excessive. Selon un autre mode de réalisation, les vergetures sont liées à une grossesse.

Selon un mode de réalisation, la peau peut être une peau jeune ou une peau mature.

### Méthode de soin

L'invention concerne également une méthode de soin cosmétique ou dermocosmétique ou cosméceutique pour protéger la peau et/ou lutter contre les rayons UV, de préférence les UVB, comprenant l'administration d'un extrait de l'invention tel que décrit ci-dessus ou d'une composition de l'invention telle que décrite ci-dessus.

L'invention concerne également une méthode de soin cosmétique, dermocosmétique ou cosméceutique de la peau pour prévenir, retarder et/ou lutter contre le photo-vieillissement de la peau, comprenant l'administration d'un extrait ou d'une composition de l'invention. La méthode de soin selon l'invention peut aussi être utile pour prévenir et/ou atténuer les rides et/ou ridules. La méthode de l'invention comprend une méthode de soin anti-âge.

L'invention concerne également une méthode de soin cosmétique, dermocosmétique ou cosméceutique pour réduire et/ou atténuer les cicatrices cutanées, comprenant l'administration d'un extrait ou d'une composition de l'invention. L'invention concerne également une méthode de soin cosmétique, dermocosmétique ou cosméceutique pour réduire et/ou limiter les vergetures, comprenant l'administration d'un extrait ou d'une composition de l'invention.

L'invention concerne également une méthode de soin cosmétique, dermocosmétique ou cosméceutique pour prévenir et/ou lutter contre l'oxydation cutanée, comprenant l'administration d'un extrait tel que défini selon l'invention en tant qu'actif ou principe ou d'une composition cosmétique telle que définie selon l'invention.

Dans la présente invention, par soin cosmétique, dermocosmétique ou cosméceutique, on entend un soin non thérapeutique.

Selon un mode de réalisation, les méthodes de soin cosmétique et dermocosmétique selon l'invention comprennent l'administration de l'extrait de l'invention ou de la composition de l'invention topiquement sur la peau du visage, des mains et/ou du corps, de préférence les zones sensibles visées par l'utilisation. Il peut s'agir, par exemple d'une simple application sur la peau ou d'une application accompagnée d'un massage de la peau avec la composition de la présente invention. Selon un mode de réalisation, il est recommandé de masser la peau jusqu'à pénétration complète de la composition dans la peau.

L'application est de préférence réalisée avec une quantité suffisante de la composition afin que toute la surface de la peau à traiter soit traitée. Il peut s'agir par exemple d'une application classique, comme une crème sur la peau. Il peut s'agir par exemple aussi d'une application pour former un masque traitant. L'application peut être réalisée sur peau préalablement mouillée ou sur peau sèche.

L'application peut être, par exemple une application quotidienne, hebdomadaire et bimensuelle. Il peut s'agir par exemple d'une application une fois par jour, deux fois par jour ou plus.

Dans un mode avantageux de réalisation des méthodes de soin non-thérapeutique selon l'invention, la composition est administrée le matin en tant que soin de jour et/ou le soir au coucher en tant que soin de nuit.

La méthode de soin cosméceutique selon l'invention inclut l'administration par voie orale d'un extrait ou d'une composition selon l'invention.

Selon un mode de réalisation, la composition de l'invention est administrée avant ou après l'exposition aux rayons UV. Selon un mode de réalisation, la composition de l'invention est administrée quotidiennement pendant au moins une semaine, de préférence au moins un mois, en prévention avant une exposition de la peau aux rayons UV. Selon un mode de réalisation, la composition de l'invention est administrée quotidiennement pendant au moins une semaine, de préférence au moins un mois, après une exposition de la peau aux rayons UV.

Dans un mode avantageux de réalisation des méthodes de soin cosmétique, dermocosmétique ou cosméceutique selon l'invention, la composition est administrée dès les premiers signes de photo-vieillissement. Selon un mode particulier de réalisation des méthodes selon l'invention, la composition est administrée en prévention de l'apparition ou dès l'apparition des premières rides et/ou ridules. Selon un autre mode particulier de réalisation des méthodes de soin cosmétique, dermocosmétique ou cosméceutique selon l'invention, la composition est administrée dès la formation d'une cicatrice.

Selon un autre mode particulier de réalisation des méthodes de soin cosmétique, dermocosmétique ou cosméceutique selon l'invention, la composition est administrée en prévention de l'apparition ou dès l'apparition des vergetures. Lors d'une grossesse, l'administration de la composition peut être réalisée dès le début de la grossesse et jusqu'après l'accouchement, par exemple jusqu'à un mois après l'accouchement.

II est bien évident que l'invention s'adresse aux mammifères en général, et plus particulièrement aux êtres humains. Selon un mode de réalisation, l'invention s'adresse aux femmes. Selon un mode de réalisation, l'invention s'adresse aux hommes. Selon un mode de réalisation, l'invention s'adresse aux adultes (personnes de plus de 18 ans). Selon un mode de réalisation, l'invention s'adresse aux adolescents (personnes entre 8 et 18 ans).

### BRÈVE DESCRIPTION DES FIGURES

La **Figure 1** est un histogramme montrant l'effet protecteur d'un extrait selon l'invention à 5%, 3% ou 1%, ou de la vitamine E à 50 µM sur des épidermes soumis à des UVB.
La **Figure 2** est un histogramme montrant le pourcentage d'inhibition de la collagénase avec un extrait selon l'invention à différentes concentrations ou avec de l'acide tannique (noté tannins) utilisé comme contrôle positif.
La **Figure 3** est un graphique montrant le diamètre d'un lattis de collagène au cours du temps, lorsqu'il n'est pas traité (témoin négatif) ou lorsqu'il est traité avec de la BSA (témoin positif) ou avec un extrait selon l'invention.

### EXEMPLES

La présente invention se comprendra mieux à la lecture des exemples suivants qui illustrent non-limitativement l'invention.

### Exemple 1 : Préparation d'un extrait selon l'invention

L'extrait selon l'invention est préparé selon le procédé décrit dans la demande de brevet internationale WO 2011/045387.

L'extrait est ensuite concentré, et trois extraits différentes sont préparés : un extrait à 1% de polyphénols, un extrait à 3% de polyphénols, et un extrait à 5% de polyphénols.

Dans les exemples qui suivent, les termes « extrait selon l'invention » se réfèrent à l'extrait préparé selon le présent exemple 1.

### Exemple 2 : Propriétés anti-inflammatoires d'un extrait selon l'invention

### Principe

L'effet protecteur d'un extrait selon l'invention vis à vis des UVB est testé sur des épidermes humains reconstitués (RHE), par évaluation de la cytotoxicité des UVB (test colorimétrique MTT) et dosage d'un marqueur de l'inflammation, l'interleukine-1 alpha (IL-1α).

### Protocole

Le produit à tester à différentes concentrations est déposé en traitement préventif à l'aide d'une micropipette à la surface des épidermes humains reconstitués (RHE) et étalé avec un petit pinceau stérile. Les épidermes sont alors incubés pendant 24h. Les épidermes sont ensuite exposés aux UVB (excepté les épidermes destinés aux témoins négatifs). Après irradiation, une deuxième application des échantillons et contrôle en traitement curatif est effectuée dans les mêmes conditions que le traitement préventif.

Les séries suivantes sont mises en oeuvre en triplicate :
- 1 série témoin négatif absolu (non irradiée),
- 1 série témoin négatif absolu (irradiée mais non traitée),
- 1 série substance à l'essai (5%, 3% et 1%), et
- 1 série témoin positif (Vit E à 50 µM).

L'effet protecteur du produit est déterminé par le test MTT et par dosage de l'interleukine IL-1α.

### 1. Test colorimétrique MTT

La prolifération cellulaire est évaluée par un test colorimétrique MTT. Le sel jaune de tétrazolium MTT (bromure de 3-(4,5-dimethylthiazol-2-yl)-2,5-diphenyl tetrazolium), lorsqu'il est réduit par la succinate déshydrogénase mitochondriale des cellules vivantes actives, forme un produit formazan bleu-violet. Le milieu passe alors du jaune au bleu-violacé. L'intensité de cette coloration est proportionnelle au nombre de cellules vivantes présentes lors du test. L'absorbance est mesurée à 540 nm.

### 2. Dosage de l'interleukine IL-1α

Le dosage de l'IL-1 α est réalisé sur les milieux de culture prélevés conformément au protocole décrit dans le coffret de dosage fourni par R&D Systems (Quantiquine Colorimetric ELISA, ref DLA50).

### Résultats

Les ratios IL-1α/MTT sont présentés dans le Tableau 1.

**Tableau 1 : Ratio IL-1α/MTT de cellules soumises à différents traitements**

| **Traitement** | **Ratio IL-1**α**/MTT** |
|---|---|
| +UVB + Extrait 5% | 129 |
| +UVB + Extrait 3% | 147 |
| +UVB + Extrait 1% | 150 |
| +UVB + Vitamine E 50 µM | 153 |
| +UVB | 205 |
| -UVB | 35 |

Le témoin absolu irradié présente un ratio IL-1α/MTT très important par rapport au témoin non irradié. Ce résultat valide le système réactif. Lorsque les épidermes sont traités avec la substance d'essai (extrait selon l'invention), on observe une diminution importante du ratio par rapport au témoin irradié.

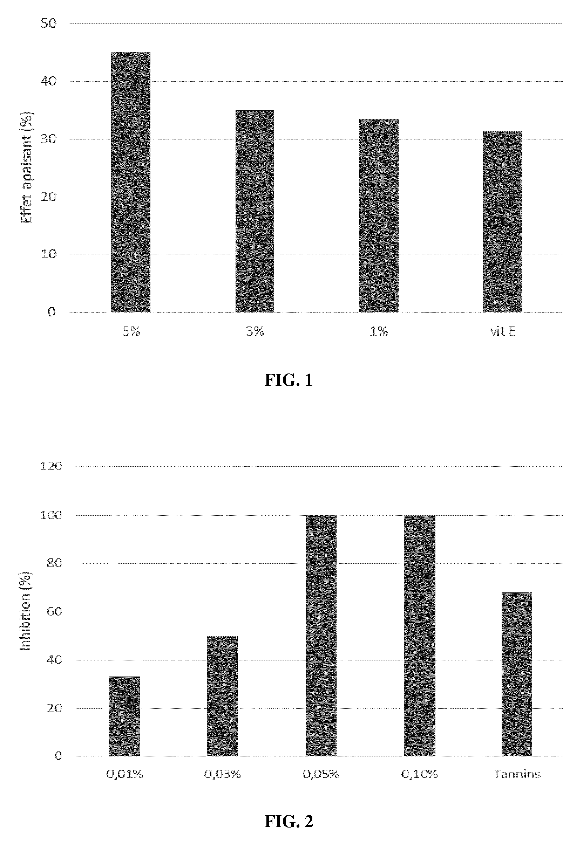

L'effet protecteur vis-à-vis des UVB est calculé en tenant compte du ratio IL-1α/MTT du témoin non irradié. Les résultats sont présentés dans la Figure 1. L'extrait selon l'invention expose un effet protecteur de 33% à une concentration de 1%, 35% à la concentration de 3% et atteint 45% avec une concentration en extrait de 5%. Le témoin vitamine E, connu pour ses propriétés anti-inflammatoires, présente quant à lui un effet protecteur de 31%.

Ainsi l'extrait selon l'invention de la présente invention possède un effet apaisant significatif.

### Exemple 3 : Activité inhibitrice d'un extrait selon l'invention sur la collagénase

### Principe

Ce test permet de déterminer l'activité enzymatique de la collagénase, enzyme dégradant les liaisons peptidiques du collagène.

Le collagène est une protéine qui a pour fonction de conférer aux tissus une résistance mécanique à l'étirement, il est notamment indispensable aux processus de cicatrisation. Il existe plusieurs type de collagène, chacun possède une structure propre et se retrouve dans des organes particuliers (le collagène de type I intervient dans la formation de la peau, des tendons, des os... le type III se retrouve au niveau du système cardiovasculaire).

Le test se base sur la dégradation par la collagénase d'un substrat (N-[3-(2-Furyl)acryloyl]-Leu-Gly-Pro-Ala ou FALGPA) qui absorbe à 345 nm. La diminution de l'absorbance correspond donc à la dégradation du substrat, elle peut être empêchée ou ralentie en présence d'inhibiteur. Des inhibiteurs connus de la collagénase sont les tanins. L'acide tannique sert donc de contrôle positif.

### Protocole

Le test est réalisé en plaque 96 puits. 5 µL d'échantillon, soit l'extrait selon l'invention à tester, soit l'acide tannique utilisé comme témoin positif à une concentration de 75 µg/mL, sont mélangés à 5 µL de collagénase à 10 U/mL (Sigma, C6885), dans un volume de 50 µL de tampon tricine 50 mM. Après une incubation de 5 min à température ambiante, le substrat FALGPA est ajouté au mélange réactionnel (90 µL à 0.25 mM, Sigma, F5135). Une cinétique de 40 min est alors démarrée, avec une lecture à 325 nm toutes les 5 min.

### Résultats

L'extrait selon l'invention présente une activité inhibitrice de la collagénase, comme le montrent les résultats de la Figure 2. En effet, l'extrait selon l'invention à 0.01% présente déjà une activité inhibitrice de la collagénase de 33%, activité inhibitrice qui atteint 100% dès 0.05% d'extrait de l'invention. De plus, cette activité inhibitrice de l'extrait de l'invention est supérieure à celle exposée par le standard de tanins.

L'extrait de l'invention présente donc une activité inhibitrice de la collagénase significative.

### Exemple 4 : Effet lissant d'un extrait selon l'invention

### Principe

Les fibroblastes humains à faible passage en culture, sont inclus dans du collagène de type 1. De par les interactions entre les molécules d'attachement des fibroblastes et le collagène, il se produit une rétraction du diamètre du lattis. Cette rétraction peut être modulée sous l'effet de produits cosmétiques. L'activation de la rétraction du lattis de collagène peut être considérée comme le marqueur d'une activité lissante. L'objectif de l'étude est donc de tester l'action d'un extrait selon l'invention sur la rétraction d'un lattis de collagène.

### Protocole

Les lattis de collagène contenant les fibroblastes sont traités avec un extrait selon l'invention à 1.25%, introduit dans le milieu de culture. Le diamètre du lattis est mesuré à 18h, 24h, 42h, 48h et 72h.

Les contrôles suivants sont inclus dans l'analyse :
- Témoin négatif : les lattis cellulaires ne subissent aucun traitement.
- Témoin positif : les lattis cellulaires sont traité avec de la BSA (albumine sérique bovine) à 2,5%.

### Résultats

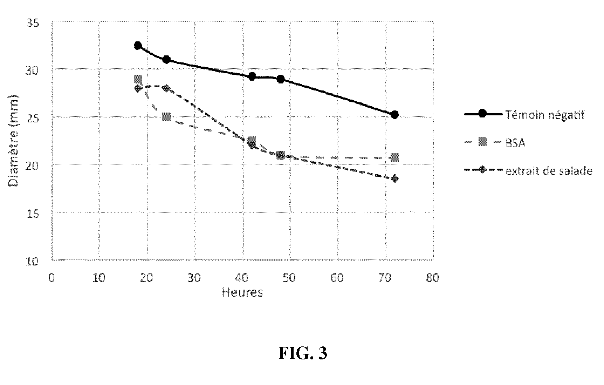

Les résultats présentés dans la Figure 3 montrent que lorsque le lattis de collagène est traité avec de la BSA, il se produit une rétraction du diamètre du lattis. Ce résultat valide le système expérimental.

Les résultats obtenus démontrent également une rétraction du diamètre du lattis lorsque celui-ci est traité avec l'extrait selon l'invention. En particulier, après 72 heures de traitement avec l'extrait de l'invention, le lattis de collagène présente une rétraction de 63%, soit une rétraction de +13.5% par rapport au témoin non traité. Le témoin positif avec la BSA présente quant à lui une rétraction de +9% par rapport au témoin non traité après la même durée de traitement. L'extrait de l'invention exhibe donc une activité 1,5 fois supérieure à celle du témoin positif.

L'extrait selon l'invention de l'invention présente ainsi une activité significative sur la contraction des fibres de collagène, c'est-à-dire une activité lissante.

## Revendications

1. Extrait de feuilles de salade, **caractérisé en ce que** ladite salade est un mélange de salades de la famille des *Asteraceae,* de la famille des *Brassicaceae* et de la famille des *Valerianaceae*, et **en ce que** l'extrait comprend de 0.01 à 25% de composés polyphénoliques en poids par rapport au poids de l'extrait sec.

2. Extrait selon la revendication **1**, dans lequel les salades de la famille des *Asteraceae* sont du genre *Lactuca*, de préférence de l'espèce *Lactuca sativa*, de préférence des variétés *capitata, crispa, longifolia* ou salades de la famille des *Brassicaceae* sont du genre *Eruca,* de préférence la variété *Eruca sativa*, et dans lequel les salades de la famille des *Valerianaceae* sont du genre *Valerianella,* de préférence la variété *Valerianella locusta*.

3. Extrait selon l'une quelconque des revendications **1** ou **2, caractérisé en ce qu'**il comprend de 1 à 30% d'acide caféoyltartarique parmi les composés polyphénoliques en poids par rapport au poids total des composés polyphénoliques.

4. Extrait selon l'une quelconque des revendications **1** à **3, caractérisé en ce qu'**il comprend de 6 à 30% d'acide caféique parmi les composés polyphénoliques en poids par rapport au poids total des composés polyphénoliques.

5. Extrait selon l'une quelconque des revendications **1** à **4, caractérisé en ce qu'**il comprend de 0 à 10% d'acide chlorogénique parmi les composés polyphénoliques en poids par rapport au poids total des composés polyphénoliques.

6. Extrait selon l'une quelconque des revendications **1** à **5**, **caractérisé en ce qu'**il comprend de 0 à 10% d'acide chicorique parmi les composés polyphénoliques en poids par rapport au poids total des composés polyphénoliques.

7. Extrait selon l'une quelconque des revendications **1** à **6,** susceptible d'être obtenu par broyage des feuilles de salade, puis hydrolyse enzymatique, puis optionnellement évaporation de l'eau.

8. Extrait selon l'une quelconque des revendications **1** à **7, caractérisé en ce qu'**il contient 10 à 99,9% en poids de matière sèche par rapport au poids total de l'extrait.

9. Composition comprenant un extrait selon l'une quelconque des revendications **1** à **8,** en association avec au moins un excipient cosmétiquement, dermocosmétiquement, cosméceutiquement ou dermatologiquement acceptable.

10. Composition selon la revendication **9, caractérisée en ce que** la composition comprend de 0.5 à 5%, de préférence de 1 à 3% d'extrait selon l'une quelconque des revendications **1** à **8** en poids par rapport au poids total de la composition.

11. Composition selon l'une des revendications **9** ou **10,** comprenant en outre au moins un autre actif en plus de l'extrait selon l'invention, de préférence choisi parmi la vitamine E, la cystéine, le sulfonate d'hydroxyquinoline, la phénantroline, le trinitrobenzène, l'acétylimidazole, ou des extraits de plantes telles que le thé, la rose ou la lavande.

12. Composition selon l'une quelconque des revendications **9** à **11, caractérisée en ce qu'**elle est formulée pour être administrée topiquement sur la peau du visage et/ou du corps, notamment sous forme de gel, lait, crème, lotion, émulsion, huile, baume, onguent ou pour être administrée oralement, notamment sous forme de comprimés, gélules, capsules, ampoules, sirops, gouttes.

13. Actif cosmétique ou dermocosmétique ou cosméceutique ou principe actif dermatologique **caractérisé en ce qu'**il comprend un extrait selon l'une quelconque des revendications **1** à **8** ou une composition selon l'une quelconque des revendications **9** à **12.**

14. Utilisation d'un extrait selon l'une quelconque des revendications **1** à **8** ou d'une composition selon l'une quelconque des revendications **9** à **12,** en tant qu'agent anti-inflammatoire et/ou apaisant et/ou lissant et/ou inhibiteur de collagénase.

15. Méthode de soin non-thérapeutique de la peau pour prévenir, retarder et/ou lutter contre le photo-vieillissement, et/ou pour prévenir et/ou atténuer les rides et/ou ridules et/ou pour réduire et/ou limiter les vergetures comprenant l'administration d'un extrait tel que défini selon l'une quelconque des revendications **1** à **8** en tant qu'actif ou principe actif ou d'une composition cosmétique telle que définie selon l'une quelconque des revendications **9** à **12.**
